# EUROPEAN PATENT APPLICATION

(11) **EP 1 269 947 A2**
(43) Date of publication of application: **02.01.2003**
(21) Application number: 02254560.2
(22) Date of filing: 28.06.2002
(51) Int. Cl.: A61F 13/02

(54) **Adhesive bandage with improved comfort and adhesion during use**

(30) Priority: 29.06.2001 US 895962
(71) Applicant: Johnson & Johnson Consumer Companies, Inc., Skillman, New Jersey 08558-9418 (US)
(72) Inventor: Falleiros, Alexandre Petrocini, Sao Jose dos Campos, Sao Paulo (BR); Aledo, Maria Aparecida de Carvalho Scamilla, Sao Jose dos Campos, Sao Paulo (BR); Serrano, Luiz Antonio, Guararema, Estado de Sao Paulo (BR); Franca, Fabio Eduardo, Jardim Leonidia, Jacarei, Sao Paulo (BR)
(74) Representative: Mercer, Christopher Paul

(57) **Abstract**

An adhesive bandage comprising a backing material, an adhesive and a wound-contacting pad which has improved comfort and improved resistance to unraveling when the body part to which it is adhered is flexed. Improved comfort and resistance to unraveling are obtained by tapering the bandage from the center region thereof to each of its opposed ends.

## Description

### Background of the Invention

### Field of the Invention

This invention relates to an adhesive bandage which provides improved comfort and resistance to release from the skin during use by the consumer. The adhesive bandage comprises a backing material, an adhesive, and a wound-contacting pad. Adhesive bandages according to the invention are designed to be more comfortable and have good adhesion especially when used to protect wounds in areas that bend, e.g., finger joints. The bandage of the invention is tapered on both sides of the absorbent pad to provide the improved comfort and resistance to displacement and unraveling from the skin of the wearer during use.

### Description of the Prior Art

Adhesives bandages are widely used to cover and protect wounds on various parts of the human body. A variety of adhesive bandage designs and shapes are commercially available to attend to different patient needs, which needs are based on, e.g., the location and severity of the wound to be protected.

It is known that fingers are one of the most frequently injured regions of the body. The bandages frequently applied to wounds on fingers have a rectangular shape or a modified rectangular shape, in which rounded edges are created by the removal of some material from the comers of the rectangle. Adhesive bandages having a modified rectangular configuration are typically made by removing material, and preferably a substantially equal amount of material, from each of the corners of an otherwise rectangular starting bandage. Typically, 3% or less of the total area of the starting rectangular bandage is removed from the corners to provide a modified rectangular bandage. The use of rectangular or modified rectangular adhesive bandages in regions of the body which are flexed during use, e.g., fingers and knuckles, sometimes results in discomfort to the wearer and frequently results in dislodgment of the bandage. Thus, there is need for an adhesive bandage which is more comfortable during use and resists dislodgment or displacement, especially during flexing, from the region of the body to which it is applied.

In co-pending Japanese Patent Application No. 332101/99, a bandage having tapered ends with slits on either side of the absorbent pad was disclosed. The slits were evidently intended to reduce stress in the area of the bandage surrounding the absorbent pad during flexion of the body part to which the bandage was applied. However, these bandages are difficult to make on a commercial scale due to the need to provide the aforementioned slits.

### Summary of the Invention

In one embodiment of the present invention, an adhesive bandage comprises:
a backing material, an adhesive, and a wound contacting pad;
said bandage having a longitudinal axis and a transverse axis, said longitudinal axis and said transverse axis intersecting each other at an angle of substantially 90°;
said bandage having an upper edge on one side of said longitudinal axis and a lower edge on the other side of said longitudinal axis;
said backing material having a first major surface and a second major surface;
said adhesive being applied to said first major surface of said backing material;
said wound contacting pad being secured to said backing material by a portion of said adhesive;
said upper edge of said bandage comprising a straight line segment which extends from a first point in said upper edge to a second point in said upper edge;
said upper edge further comprising a first pair of substantially identical arcuate segments each of said first arcuate segments having a first radius of curvature, one of said arcuate segments extending laterally from said first point in said upper edge to a first point of inflection in said upper edge and the other of said arcuate segments extending from said second point in said upper edge to a second point of inflection in said upper edge;
said upper edge further comprising a second pair of substantially identical arcuate segments, each of said second arcuate segments having a second radius of curvature, one of said second arcuate segments extending from said first point of inflection to a first end point and the other of the said second arcuate segments extending from said second point of inflection to a second end point;
said lower edge of said bandage comprising a straight line segment which extends from a first point in said lower edge to a second point in said lower edge;
said lower edge further comprising a first pair of substantially identical arcuate segments, each of said arcuate segments having a first radius of curvature, one of said arcuate segments extending laterally from said first point in said lower edge to a first point of inflection in said lower edge, and the other of said arcuate segments extending from said second point in said lower edge to a second point of inflection in said lower edge;
said lower edge further comprising a second pair of substantially identical arcuate segments, each of said second arcuate segments having a second radius of curvature, one of said second arcuate segments extending from said first point of inflection in said lower edge to said first end point and the other of the said second arcuate segments extending from said second point of inflection in said lower edge to said second end point;
said upper edge and said lower edge, when joined respectively at said first and second end points, defining the periphery of said bandage;
the area of said bandage lying within said periphery ranging from about 65% to about 95% of the area of a rectangle in which said bandage may be inscribed.

When the bandage according to this embodiment is so inscribed in said rectangle, the straight line segments comprising the upper and lower edges of the bandage are coincident with the upper and lower edge of the rectangle in which said bandage may be inscribed and the first and second side edges of said rectangle in which said bandage may be inscribed pass, respectively, through said first end point and said second end point.

### Detailed Description of the Invention

The bandage of the present invention comprises a generally flexible backing material. Any conventional backing material known for use in adhesive bandages may be utilized. Suitable backing materials include, but are not limited to, polyolefin films, such as polyethylene and polypropylene; polyvinylchloride films; ethylene-vinyl acetate films; woven, nonwoven or knitted fabrics; and the like.

A woven backing material which may be used comprises a polyester yarn such as a polyethylene terephthalate or a polybutylene terephthalate yarn in the warp direction and a polyamide yarn (such as nylon 6 or nylon 6,6) in the fill direction. Another useful woven backing material comprises a polyethylene terephthalate yarn in the warp direction and a polybutylene terephthalate yarn in the fill direction.

One major surface of the backing material has an adhesive applied thereto. A portion of the adhesive serves to secure a wound-contacting pad to the backing while the remaining portion of the adhesive serves to secure the bandage to the skin during use. Any conventional adhesives known for use in adhesive bandages may be utilized. The adhesives may be hot melt adhesives. Examples of suitable adhesives include, but are not limited to, those based on blends of styrenic block copolymers and tackifying resins. Useful adhesives are HL-1491 available from HB-Fuller Co. (St. Paul MN); H-2543 available from ATO-Findley (Wawatausa, WI); and 34-5534 available from National Starch & Chemical (Bridgewater, NJ). Ethylene copolymers, including ethylene-vinyl acetate copolymers, may also be used.

Suitable adhesives also include acrylic based, dextrin based, and urethane based adhesives as well as natural and synthetic elastomers. The adhesives may also include amorphous polyolefins, including amorphous polypropylene, such as HL-1308 available from HB Fuller or Rextac RT 2373 available from Huntsman (Odesssa, TX). The adhesive may be compounded with Kraton® brand synthetic rubber and the like, or natural rubber with a tackifier and antioxidant and other processing aids.

Hot melt adhesives can be applied in the molten state, e.g., by spraying, coating, or other known methods. The amount of adhesive typically applied to the backing is well-known to those skilled in the art. Typically, the adhesive coating weight may vary from about 20 grams per square meter ("gsm") to about 100 gsm.

A wound-contacting pad is secured to the central portion of the backing to cushion the wound and protect the wound from contamination by dirt. The wound contacting pad comprises at least one layer of a body-fluid absorbent material. In a preferred embodiment, said at least one layer of body-fluid absorbent material is covered with a porous covering layer. The porous covering layer is preferably an open-mesh polymeric netting, e.g., a polypropylene netting.

In most instances, the wound-contacting layer is centered on the backing material from end to end of the bandage. If desired, the wound contacting pad may be offset from center so as to be closer to one end of the bandage than the other. The absorbent layer may be made from various body fluid absorbent materials including rayon; natural fibers, such as, but not limited to, cotton and wood pulp; synthetic fibers, such as, but not limited to, polyester, polyamide, and polyolefin fibers. Polyesteramide, polyetheramide and the like fibers may also be used. Blends of two or more fibers may be used as well. The fibers may be bicomponent fibers. For example, the fibers may have a core of one polymer, and a sheath of a different polymer. Alternatively, the two polymers comprising the bicomponent fiber may be in a "side-by-side" configuration.

The fibers comprising the absorbent layer of the wound-contacting pad may, if desired, be bonded so as to increase structural integrity. For example, where the wound-contacting pad comprises heat fusible fibers, the pad may be bonded by the application of heat. Alternatively, and especially where the absorbent layer is made of fibers which are not heat-sealable or are not easily heat-sealable, the pad may be bonded by the application thereto of a polymeric adhesive, either solvent based or aqueous based, followed by drying.

The fibers comprising the absorbent layer of the wound-contacting pad typically have deniers ranging from about 1-10, but other deniers may be used if desired. The fibers may, if desired, be hollow fibers.

The basis weight for the absorbent layer is not critical, but typically may range from 0.003 g/cm² to 0.015 g/cm². The size of the wound-contacting pad may vary depending on the wound to be protected or treated.

The bandage of the present invention has a length (l) in its longitudinal direction, and a width (w) in its transverse direction. The width of the bandage at its widest part typically ranges from about 10 mm to about 30 mm. Preferably, the ratio of the maximum length to the maximum width of the bandage of this invention is from about 2.5 to about 5. The maximum width of the bandage occurs in a generally central region thereof, inwardly of its opposed ends. The bandage is tapered from its central region toward each of its ends.

In order to obtain an acceptable balance of adhesion to the skin and protection of the wound, the length of the wound contacting pad is typically from about 20 percent to about 70 percent, preferably from about 25 percent to about 50 percent, of the overall length of the backing material. As is known in the art, the width of the wound-contacting pad may be substantially equal to or less than the width of the backing material.

The bandage of the present invention may be inscribed in a rectangle whose length corresponds to the length, l, of the bandage and whose width, w., corresponds to the maximum width of the bandage. In the bandage according to the present invention, the maximum width of the bandage occurs in a generally central region between its tapered ends. When so inscribed in said rectangle, the bandage of the present invention occupies an area of from about 65 percent to about 95 percent of the area of the rectangle in which the bandage may be inscribed. This is accomplished by the removal of a portion of the backing material from each of its corners to form tapered portions of the bandage. Preferably, the bandage of the present invention occupies an area of from about 70 percent to about 93 percent of the area of the rectangle in which the bandage may be inscribed. More preferably, the bandage of the present invention occupies from about 75 percent to about 91 percent of the area of the rectangle in which the bandage may be inscribed.

Bandages in accordance with the present invention are able to more uniformly distribute the stresses applied to them during use, thus providing improved comfort and skin adhesion.

Examples are set forth below to further illustrate the nature of the invention and the manner of carrying it out. However, the invention should not be considered as being limited to the details thereof.

### Brief Description of the Drawings

Descriptions of the Drawing Figures follow. The descriptions are intended to illustrate various aspects of the invention, but should not be construed as limiting the scope of the invention.

Figure 1 is a top plan view of a bandage of the present invention.

Figure 2 is a longitudinal section taken along longitudinal axis, L-L, of Figure 1 and showing a wound contacting pad 25 comprising an absorbent layer 27 and a porous covering layer 28.

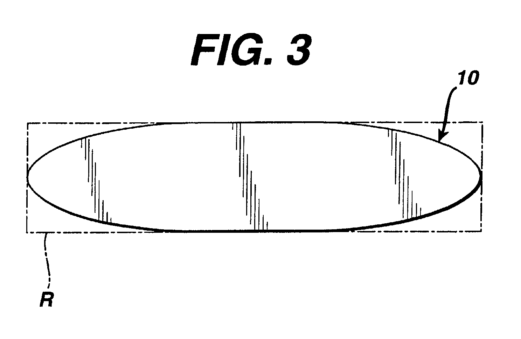

Figure 3 shows one embodiment of the adhesive bandage of the present invention inscribed within a rectangle, R, the length of said bandage being substantially equivalent to the length of said rectangle and the maximum width of said bandage being substantially equal to the width of said rectangle.

Figure 4 is a top plan view of a substantially rectangular adhesive bandage known in the prior art.

Figure 5 is a top plan view of a typical modified rectangular adhesive bandage known in the prior art.

Figures 6-8 are plan views of bandages in accordance with the teachings of the present invention, each of said bandages occupying an area which is less than the area of the rectangle in which it may be inscribed.

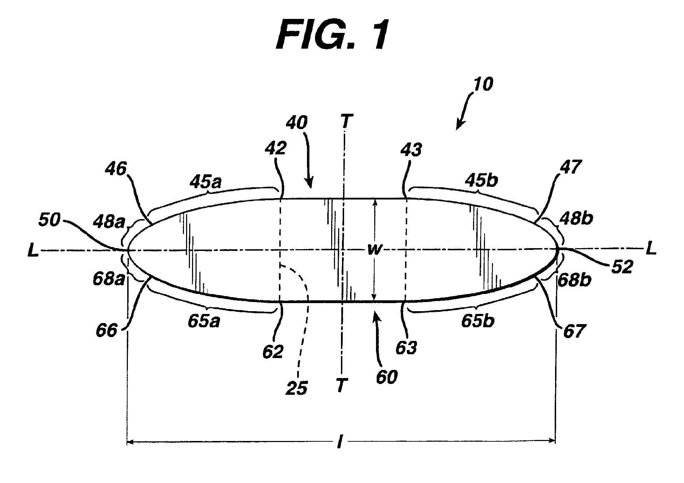

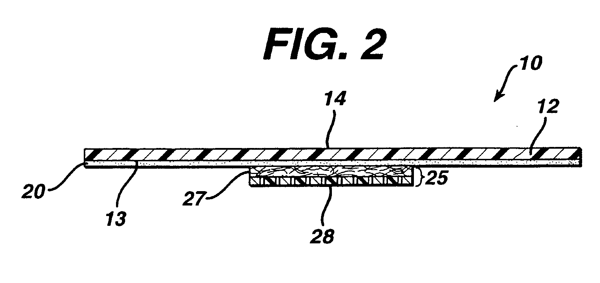

As is seen in the drawings, especially Figures 1 and 2, bandage 10 comprises a backing material 12, an adhesive 20, and a wound-contacting pad 25. The bandage has a longitudinal axis, L-L, and a transverse axis, T-T, the longitudinal axis and the transverse axis intersecting each other at an angle of substantially 90°. The bandage has an upper edge 40 on one side of the longitudinal axis and a lower edge 60 on the other side of the longitudinal axis. The backing material has a first major surface 13 and a second major surface 14. The adhesive 20 is applied to the first major surface 13 of the backing material 12. The wound-contacting pad 25 is secured to the backing material 12 by a portion of the adhesive 20.

The upper edge 40 of the bandage has a first straight line segment which extends from a first point 42 in the upper edge to a second point 43 in the upper edge. The upper edge 40 further includes a first pair of substantially identical arcuate segments 45 a and 45 b, each having a first radius of curvature. One of the arcuate segments, 45 a, extends laterally from first point 42 to a first point of inflection 46 in the upper edge 40 and the other of the arcuate segments, 45 b extends from second point 43 to a second point of inflection 47 in the upper edge 40.

The upper edge 40 further includes a second pair of substantially identical arcuate segments, 48 a and 48 b. Each of the second arcuate segments has a second radius of curvature. One of the second arcuate segments, 48 a, extends from the first point of inflection (46) to a first end point 50 and the other of the second arcuate segments, 48 b, extends from the second point of inflection 47 to a second end point 52.

The lower edge 60 of the bandage has a first straight line segment which extends from a first point 62 in the lower edge to a second point 63 in the lower edge. The lower edge 60 further includes a first pair of substantially identical arcuate segments 65 a and 65 b, each having a first radius of curvature. One of the arcuate segments, 65 a, extends laterally from first point 62 in the lower edge to a first point of inflection (66) in the lower edge 60. The other arcuate segment, 65 b, extends from second point 63 in the lower edge 60 to a second point of inflection 67 in the lower edge 60.

The lower edge 60 further includes a second pair of substantially identical arcuate segments 68 a and 68 b, each of the second arcuate segments having a second radius of curvature. One of the second arcuate segments, 68 a, extends from the first point of inflection 66 in the lower edge 60 to the first end point 50. The other of the second arcuate segments, 68 b, extends from second point of inflection 67 in the lower edge 60 to the second end point 52.

The upper edge 40 and the lower edge 60, when joined respectively at the first and second end points 50 and 52, define the periphery of the bandage. The area of the inventive bandage lying within said periphery ranges from about 65% to about 95% of the area of a rectangle in which the bandage may be inscribed.

Preferably, the length of the aforementioned straight line segments constitutes less than about 50% of the length, I, of the bandage, while the total length of the two adjacent tapered sections constitutes more than 50%. Preferably, the length of the straight line segments constitutes less than 40% of the length, l, of the bandage.

Referring to FIG. 1, it is preferable that adhesive bandages in accordance with the invention be symmetrical when folded around their longitudinal axis, L-L, and when folded around their transverse axis, T-T.

### Examples

Finite elemental analysis was performed on various bandage embodiments to determine regions of the bandage that undergo stress, due to flexion of body parts, during use. These stressed regions result in discomfort and poor skin adhesion during use. The study simulates the stress to which an adhesive bandage is exposed when it is applied to a joint, such as a knuckle, a knee, or an elbow, and flexed during use. In this simulation, the bandage is applied on a substratum with elastic characteristics similar to those of human skin. The substratum is then elongated in the longitudinal and transverse direction exposing the product to stress.

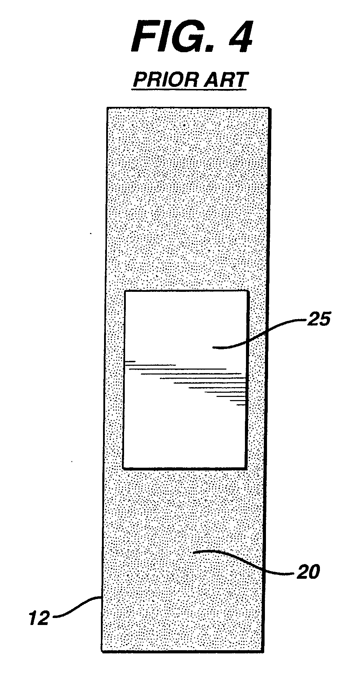

Bandage designs which were subjected to finite elemental analysis are shown in Figures 4, 5, 6, 7 and 8. The bandage of Figure 4 was a substantially rectangular bandage measuring 19mm wide and 76mm long. The bandage contains a wound-contacting pad which is 14 mm wide and 25 mm long and which is centered from end-to-end and from side-to-side of the bandage.

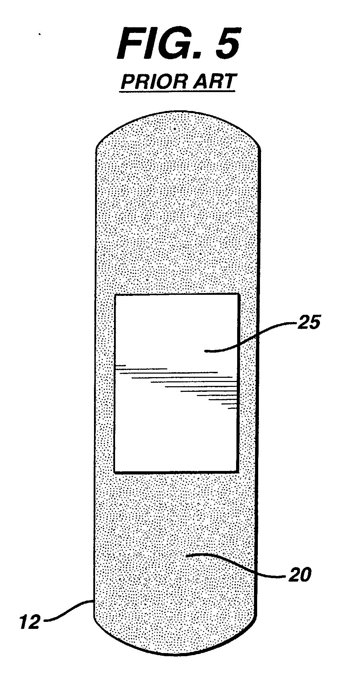

The adhesive bandages of FIGS. 5-8 have the same overall length, the same overall width, the same adhesive and coating weight, and the same wound-contacting pad dimensions and location as the adhesive bandage shown in FIG. 4. The backings of the adhesive bandages of FIGS. 5-8, however, occupy only a percentage of the area of the rectangle in which they may be inscribed. Further, as is preferred, the backings of the bandages of FIGS. 5-8 are symmetrical when folded around their longitudinal and transverse axes.

The adhesive bandage shown in FIG. 5 occupied an area which was about 97% of the area of the rectangle in which it could be inscribed. This adhesive bandage is representative of the modified rectangular bandage known in the prior art.

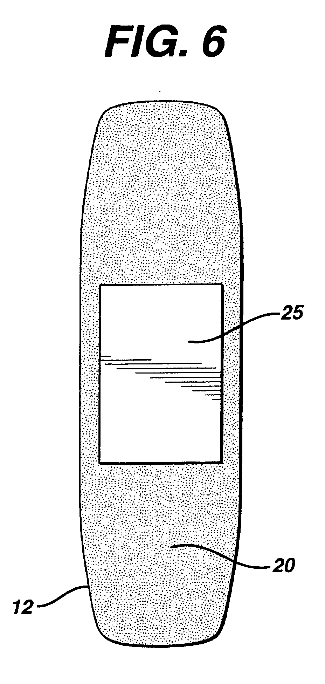

The adhesive bandage shown in FIG. 6 occupied an area which was about 91% of the area of the rectangle in which it could be inscribed.

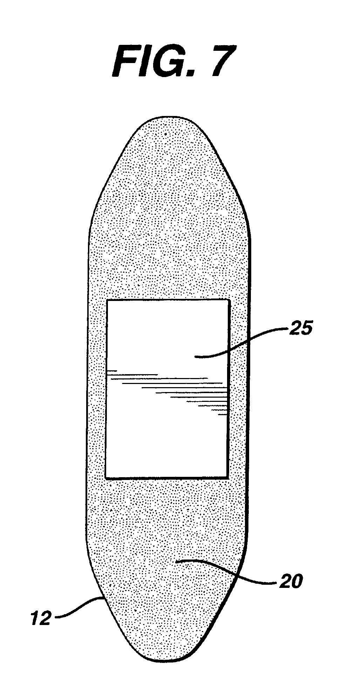

The adhesive bandage shown in FIG. 7 occupied an area which is about 80% of the area of the rectangle in which it could be inscribed.

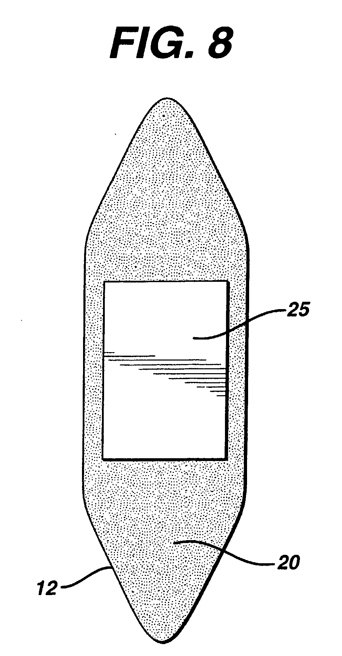

The adhesive bandage shown in FIG. 8 occupied an area which is about 70% of the area of the rectangle in which it could be inscribed.

Commercially available ANSYS Finite Elemental Analysis Software was utilized for the analysis of the various adhesive bandage embodiments of Figures 4-8.

For the analysis, models of the bandages were presented to the software program as being perfectly bonded to a 1 mm thick substrate simulating skin. The following modulus of elasticity coefficients were utilized in the software program: skin-simulating substrate: 1.75 MPa; bandage backing: 12.4 MPa; absorbent layer of wound-contacting pad: 11.4 MPa; and porous netting cover layer of wound-contacting pad: 1 MPa. The bandage models were discretized into meshes of quadratic solid elements with 20 nodes and 3 displacement degrees of freedom per node. The number of finite elements utilized per bandage model ranged from 5,056 to 5,631.
The bandage models were stressed with an 8 mm displacement force in the transverse direction and 12 mm displacement force in the longitudinal direction. A nonlinear iterative analysis with finite deformations was performed on each bandage model. The stress in the X (transverse) and Y (longitudinal) direction for one quadrant of each adhesive bandage was measured using finite elemental analysis, and the Von Mises stress was calculated.

The following predictions were made from a consideration of the aforementioned calculated Von Mises stress values.

The substantially rectangular adhesive bandage of Figure 4 was predicted to be uncomfortable in use and to have little resistance to unraveling or displacement from the skin in regions where it would be subjected to flexing and bending of a body part, e.g., a knuckle. Such results are consistent with actual experience with substantially rectangular adhesive bandages of the prior art.

The results of the analysis of the modified rectangular bandage of Figure 5 suggest that it would be somewhat more comfortable during use and would have somewhat improved resistance to unraveling when compared to the adhesive bandage of Figure 4. These improvements, however, were relatively minimal and, again, were consistent with actual experience with modified rectangular bandages of the prior art.

The results of the analysis of the adhesive bandage of Figure 6, an adhesive bandage in accordance with the teachings of the present invention, suggest that this bandage would be significantly more comfortable during use and would have significantly increased resistance to unraveling and displacement during use than either of the prior art bandages of Figures 4 and 5.

The results of the analysis of the adhesive bandage of Figure 7, another adhesive bandage in accordance with the teachings of the present invention, suggest that this bandage would be even more comfortable during use and would have better resistance to unraveling and displacement during use than the bandage of Figure 6.

Finally, the results of the analysis of the adhesive bandage of Figure 8, a third adhesive bandage in accordance with the teachings of the present invention, suggest that this bandage would have a degree of comfort during use and a degree of resistance to unraveling and displacement during use which are comparable to the comfort and resistance to unraveling suggested for the adhesive bandage of Figure 7.

## Claims

1. An adhesive bandage comprising:
a backing material, an adhesive, and a wound contacting pad;
said bandage having a longitudinal axis and a transverse axis, said longitudinal axis and said transverse axis intersecting each other at an angle of substantially 90°;
said bandage having an upper edge on one side of said longitudinal axis and a lower edge on the other side of said longitudinal axis;
said backing material having a first major surface and a second major surface;
said adhesive being applied to said first major surface of said backing material;
said wound contacting pad being secured to said backing material by a portion of said adhesive;
said upper edge of said bandage comprising a straight line segment which extends from a first point in said upper edge to a second point in said upper edge;
said upper edge further comprising a first pair of substantially identical arcuate segments, each of said first arcuate segments having a first radius of curvature, one of said arcuate segments extending laterally from said first point in said upper edge to a first point of inflection in said upper edge and the other of said arcuate segments extending from said second point in said upper edge to a second point of inflection in said upper edge;
said upper edge further comprising a second pair of substantially identical arcuate segments, each of said second arcuate segments having a second radius of curvature, one of said second arcuate segments extending from said first point of inflection to a first end point and the other of the said second arcuate segments extending from said second point of inflection to a second end point;
said lower edge of said bandage comprising a straight line segment which extends from a first point in said lower edge to a second point in said lower edge;
said lower edge further comprising a first pair of substantially identical arcuate segments, each of said arcuate segments having a first radius of curvature, one of said arcuate segments extending laterally from said first point in said lower edge to a first point of inflection in said lower edge and the other of said arcuate segments extending from said second point in said lower edge to a second point of inflection in said lower edge;
said lower edge further comprising a second pair of substantially identical arcuate segments, each of said second arcuate segments having a second radius of curvature, one of said second arcuate segments extending from said first point of inflection in said lower edge to said first end point and the other of the said second arcuate segments extending from said second point of inflection in said lower edge to said second end point;
said upper edge and said lower edge, when joined respectively at said first and second end points, defining the periphery of said bandage;
the area of said bandage lying within said periphery ranging from 65% to 95% of the area of a rectangle in which said bandage may be inscribed.

2. An adhesive bandage according to Claim 1 wherein said wound covering layer comprises at least one layer of body-fluid absorbent material.

3. An adhesive bandage according to Claim 2 wherein said at least one layer is covered with a porous covering layer.

4. An adhesive bandage according to Claim 3 wherein said porous covering layer is an open-mesh polymer netting.

5. An adhesive bandage according to Claim 1 wherein the area of said bandage lying within said periphery ranges from 70% to 93%, preferably from 75% to 91%, most preferably about 80%, of the area of a rectangle in which said bandage may be inscribed.

6. An adhesive bandage according to Claim 1 wherein said wound-contacting pad is centered from end-to-end of the bandage.

7. An adhesive bandage according to Claim 1 wherein said wound-contacting pad is closer to one end of said bandage than to the other end of said bandage.

8. An adhesive bandage according to Claim 1 wherein said backing material is symmetrical with respect to said longitudinal axis.

9. An adhesive bandage according to Claim 1 wherein said backing material is symmetrical with respect to said transverse axis.

10. An adhesive bandage according to Claim 1 wherein said backing material is symmetrical with respect to said longitudinal axis and said transverse axis.
